# EUROPEAN PATENT SPECIFICATION

(11) **EP 1 940 233 B1**
(45) Date of publication and mention of the grant of the patent: **13.08.2014**
(21) Application number: 06807232.1
(22) Date of filing: 13.10.2006
(51) Int. Cl.: A01P 7/04, A01P 7/00, A01N 59/04, A01N 25/04, A01N 59/00

(54) **PEDICULICIDAL PROCESS AND COMPOSITION**
PEDICULIZIDVERFAHREN UND ZUSAMMENSETZUNG
PROCÉDURE ET COMPOSITION PÉDICULICIDES

(30) Priority: 17.10.2005 FR 0510553
(43) Date of publication of application: 09.07.2008
(73) Proprietor: SOLVAY SA, 1120 Bruxelles (BE)
(72) Inventor: PALANGIE, Nicolas, F-60880 Le Meux (FR); PASCAL, Jean-Philippe, F-54000 Nancy (FR)
(74) Representative: Gilliard, Pierre
(86) International application number: PCT/EP2006/067367
(87) International publication number: WO 2007/045608

(56) References cited:
- WO-A-03/045152
- WO-A-2004/028250
- WO-A-2004/056184
- WO-A-2005/025317

## Description

The invention relates to a pediculicidal process and to a pediculicidal composition, that is to say a process and a composition which are effective in combating the arthropod Pediculus humanus and/or its nits. It also relates to a method for killing the Pediculus humanus arthropods growing on human head hair using such a composition.

The lice growing on the human body (Pediculus humanus) present a known public health problem. These lice comprise, in a known way, Pediculus humanus capitis (head louse), indeed even Pediculus humanus corporis (body louse), and Phthirus pubis (pubic louse). Although the infestation of children by lice has been declining in recent decades, in France alone more than two million children still have to be treated each year, which is a worry to families and officials of primary schools concerned. The struggle against lice is made particularly complex in that the eggs of lice are protected by an extremely watertight casing and are firmly attached to human hair. This casing exhibits only a few very small openings, known as aeropiles, which are situated on an operculum at the top of the nit, allowing the nit to breathe, these aeropiles constituting the only passages which allow a pediculicide to penetrate into the nit. During the hatching of the nits, the operculum comprising the aeropiles opens to allow the nymph, which climbs out of the watertight protective casing, to pass.

It is known to combat lice and their nits using neurotoxic substances, such as pyrethrins or synthetic pyrethroids (permethrin, allethrin, bioallethrin, D-phenothrin, and the like), organophosphorus compounds, such as malathion, carbamates (carbaryl, and the like) or piperonyl butoxide. However, it appears that these substances are gradually losing their effectiveness following the appearance of strains of Pediculus humanus which are resistant to them. Furthermore, their use in the vicinity of the human body can result in skin irritation and risks due to their neurotoxicity when used repeatedly. This is because these neurotoxic substances act on the nervous system of the louse and are also toxic to man. Some of these products are even suspected of being carcinogenic, indeed even teratogenic. This toxicity is a particular concern in the case of children, for whom repeated infestations by lice often require repeated treatments.

WO05/025317 discloses an aqueous parasiticidal suspension comprising silica and sodium bicarbonate and a method for controlling parasites on animals, in particular farm animals. Among cited parasites whcich can be controlled by said suspension, acarids such as red poultry lice are cited. However, a process or a composition comprising more than 60% bicarbonate for combating the specific louse Pediculus humanus (that is an insect) is not disclosed. In addition, the composition disclosed is not in form of a gel. The document discloses one result in short time against acarid parasite (Dermanyssus gallinae) at Example 3, Table 1 page 7, with an aqueous suspension (not a gel) made of 20 kg of a dry mixture of Sodium bicarbonate 85% and different Silica 15%; and 25 kg water (i.e.: 37% Sodium bicarbonate, 7% Silica, 56% water), the said aqueous suspension is applied on a plate and let dried 24h before being put into contact with parasite, showing 0 to 5% (column 2) of parasite mortality after 1 hour of contact.

A composition based on saccharide and on silica which is effective in combating lice is disclosed in WO03/045152. However, this composition is relatively expensive and its pediculicidal action is fairly slow.

WO 04/056184 discloses the use of a powder comprising more than 40 wt. % of sodium bicarbonate and silica for controlling parasites, in particular acarids, insects and fungi, in cereal storage.

WO 04/028250 discloses the use of a composition comprising specific silicon containing particles to combat or repel several types of pests, among which Pediculus spp. (lice) are cited. Said composition can be in form of a gel and can be used in cosmetic products, such as shampoos or shower gels. However, it does not contain any alkali metal bicarbonate.

The invention is targeted at providing a novel pediculicidal process to which the lice are not resistant and which is economic and a composition harmless to man.

Consequently, the invention relates to a pediculicidal composition comprising at least 60% by weight of alkali metal bicarbonate for use in the elimination of the louse Pediculus humanus from human hair head.

In the pediculicidal composition according to the invention, the alkali metal bicarbonate can, for example, be bicarbonate in the strict sense, such as potassium bicarbonate or sodium bicarbonate. In this account, however, it also covers compound salts, such as alkali metal sesquicarbonates (for example trona), which comprise bicarbonate. Sodium bicarbonate, potassium bicarbonate or trona are especially well suited. Bicarbonates in the strict sense are recommended. Potassium bicarbonate or sodium bicarbonate, more particularly sodium bicarbonate, is preferred. The composition comprises at least 60%, advantageously 70%, preferably 80%, more preferably still 85%, by weight of alkali metal bicarbonate.

The bicarbonate advantageously comprises particles having a particle size distribution such that at least 50%, advantageously 75%, preferably 90%, of the particles have a mean diameter of less than 100 µm, preferably 70 µm. The mean diameter is advantageously less than 50 µm, preferably 40 µm. The diameters are measured according to Standard ASTM C - 690 - 1992. The remainder of the bicarbonate, which is not in the form of particles, can then, for example, be in the dissolved form. In addition to its chemical action against Pediculus humanus, the inventors consider that the alkali metal bicarbonate has a mechanical action which is more effective when it has such particle sizes.

In a preferred embodiment of the pediculicidal composition according to the invention, the composition additionally comprises at least 1%, preferably 5%, of silica. It is recommended that it should not comprise more than 20%, preferably 15%, thereof. It has been observed that the presence of silica in the powder synergistically increases the pediculicidal effects of the alkali metal bicarbonate. Furthermore, as the powder comprises high concentrations of bicarbonate, the presence of silica improves the flow of the suspension and thus favours its homogeneous application. It is recommended that the silica should be in the amorphous (and noncrystalline) form in order to be tolerated by the human body, in particular in the event of inhalation.

It is preferable for the silica to be in the form of very fine particles having a high specific surface, for example of greater than 200 m²/g, preferably 400 m²/g, measured according to Standard ISO 5794-1, annex D. In an advantageous embodiment of the invention, the silica is in the form of particles having a mean diameter of less than 10 µm. The mean diameter is measured according to Standard ASTM C -690 - 1992.

The bicarbonate/silica mixture must be as homogeneous as possible. However, it has been observed that, in certain circumstances, in particular when the mixing is carried out in ploughshare mixers, which is advantageous, an excessively long mixing time can result in a loss in effectiveness of the subsequent aqueous suspension. Generally, it is recommended to handle the alkali metal bicarbonate/silica mixture so as to fluidize it. This fluidization takes place in a ploughshare mixer when the mixture falls back in the mixer following the rotation of the ploughshare.

In particular when it is in the form of particles having the most advantageous particle sizes, the bicarbonate has its own parasiticidal action and its presence in the composition does not have a function of diluting or carrying the silica.

Alkali metal bicarbonates and silica are substances harmless to man and animals. In the case of sodium bicarbonate and potassium bicarbonate, they are even permitted by various bodies (such as the Food and Drug Administration in the United States and the EEC) in human food. In the pediculicidal composition according to the invention, the powder does not require any other substance active against Pediculus humanus, such as neurotoxic pediculicides. It is recommended that it should be devoid of any neurotoxic substance. It is even preferable for it to be completely devoid of any other active pediculicidal substance.

In the pediculicidal composition according to the invention, the composition can be in various formulation forms, such as powder, cream, gel, aqueous suspension or aqueous solution.

In a first alternative form of the pediculicidal composition according to the invention, the composition is in the form of an aqueous suspension. This formulation form is advantageous for the formulating of shampoos. In this alternative form, it is recommended for the aqueous suspension, which may not be confused with an aqueous solution, to comprise an amount of alkali metal bicarbonate which exceeds its solubility limit at ambient temperature. It has been observed that, in this case, the particle size of the bicarbonate particles in the suspension is more stable over time.

In a second alternative form of the pediculicidal composition according to the invention, which is preferred, the composition is in the gel form. Gels are compositions comprising at least two components, generally a solid dispersed in the colloidal form in a liquid phase. The dispersed particles form spatial networks stabilized by means of Van der Waals' forces. In hydrogels, the liquid phase is water. They can also comprise gelling polymers, such as polysaccharides, pectin, xanthan, and the like. In this alternative form, it is preferable for the composition to comprise silica. When the composition comprises silica, it is particularly easy to produce a gel by simple addition of water to the bicarbonate/silica mixture. It is then preferable for the composition to be devoid of gelling polymers. This alternative form can be easily employed in particular on human head hair as prolonged contact between Pediculus humanus and the composition is provided by the consistency of the gel, which makes it possible to avoid recourse to coverings, such as caps. Finally, such a composition is completely harmless, which is advantageous in treating the lice present on young children as the latter frequently touch their head hair and may subsequently bring their hand to their mouth.

The invention thus also relates to a composition in the gel form comprising from 35 to 55%, preferably from 40 to 50%, by weight of alkali metal bicarbonate particles, from 10 to 30%, preferably from 15 to 25%, of silica and from 30 to 50%, preferably from 35 to 45%, of water.

It is advantageous for the composition not to comprise more than 10%, preferably 5%, in particular 1%, of organic matter, such as a gelling polymer, for example xanthan gum. Although in some cases it is recommended for it to comprise at least 0.1 % by weight of such a gelling agent (for example xanthan gum) in order to improve the rheological properties of the gel, it is particularly advantageous for the composition to be completely inorganic.

The composition in the gel form according to the invention can be obtained very simply by simple mixing of the constituents.

Such a gel, by virtue of its simplicity of use and of its consistency, has numerous applications. It has appeared particularly useful in combating parasites in general (such as, for example, ticks, lice, acarids) as parasiticidal composition. However, it is particularly suitable for combating Pediculus humanus as pediculicidal composition by virtue of its harmlessness to man and its excellent effectiveness against the nits of this parasite. The pediculicidal composition is then advantageously devoid of any other pediculicidal substance. Without wishing to be committed to a theoretical explanation, the inventors suspect that the composition according to the invention results in blocking of the aeropiles, which prevents the nymphs from hatching.

The ingredients of the composition according to the invention are preferably, insofar as possible, in accordance with those of the composition used in the specific alternative forms and embodiments of the process according to the invention. For example, the particulate bicarbonate, which is advantageously sodium bicarbonate, is preferably in the form of particles having a particle size such that at least 50%, advantageously 75%, preferably 90%, of the particles have a mean diameter of less than 100 µm, preferably 70 µm. The gel then has a very smooth consistency which is particularly pleasant to the touch and its parasiticidal effectiveness in general but in particular its pediculicidal effectiveness is thereby improved.

The composition and the process according to the invention are particularly effective in combating Pediculus humanus infesting substrates such as human body linen, bedding and in particular pillows, cleaning materials, such as towels, human hair in general and in particular human head hair. In particular, the invention relates to a process for combating the louse pediculus humanus infesting body linen, bedding and in particular pillows, and cleaning materials, such as towels, according to which the louse is brought into contact with a composition according the invention. As the composition and the process according to the invention employ nontoxic substances with a rather mild action, they are advantageously intended to combat infestations from the beginning of their development, in particular on human head hair.

Advantageously, the head hair is brought into contact with the pediculicidal composition for at least 1 hour, preferably 4 hours, more preferably 8 hours, the operation of bringing into contact for at least 1 hour, preferably 4 hours, more preferably 8 hours, subsequently advantageously being repeated at the earliest 2 days later. However, it is recommended for the second operation of bringing into contact to take place less than 10 days, preferably one week, after the first. Additional operations of bringing into contact may be provided, if necessary. Care will particularly be taken that the composition is applied homogeneously to the hair while definitely reaching the base of the head hair, the nits generally being attached to the part of the individual head hair closest to the scalp.

Consequently the invention relates also to a composition according the invention for eliminating the louse Pediculus humanus from human head hair, by bringing the composition into contact with the head hair for at least 1 hour, the operation of bringing into contact for at least I hour subsequently being repeated at the earliest two days later

The following examples serve to illustrate the invention.

### Example 1

Strains of the louse Pediculus humanus suited to the rabbit were selected. Right from their birth, the young hatched lice, placed on pieces of material simulating the hair, were set down once daily on the shaven stomach of a rabbit in order to gorge themselves with blood. Right after they had finished feeding, they were placed in an oven maintained at 29°C and 70 - 80% relative humidity. Four batches of 25 lice aged between 3 and 7 days after hatching (nymphs), on the one hand, and aged between 15 and 16 days after hatching (adults), on the other hand, were then brought into contact for 1 hour with a powder comprising 85% by weight of sodium bicarbonate Bicar® 0/4, composed of particles having a particle size such that 75% of the particles have a mean diameter of less than 65 µm, and 15% by weight of silica Sipernat® 50S manufactured by Degussa. After the treatment, the lice were washed. 16 hours after the treatment, all the lice fed and stored normally were dead, whether nymphs or adults. In comparison, lice only washed, not having been brought into contact with the powder, showed a mortality of approximately 9%.

### Example 2

In Example 2, two batches of 50 lice, treated in the same way as in Example 1, were brought in contact for 3h with a gel consisting of 43% of bicarbonate (composed of particles having a particle size such that 75% of the particles have a mean diameter of less than 65 µm), 19% of silica Sipernat®50S and 38% water. Already 1h after the treatment, 98% of the lice were dead. When the contact time was increased from 3h to 6h, 1h after the treatment, 100% of the lice were dead.

### Example 3

In example 3, lice nits, laid between 1 and 4 days previously, were taken. The nits were brought into contact for 8 hours with a powder analogous to that of Example 1. The nits were subsequently washed and placed in the oven (29°C, 70

## Claims

1. Pediculicidal composition comprising at least 60% by weight of alkali metal bicarbonate for use in the elimination of the louse Pediculus humanus from human head hair.

2. Pedicucidal composition according to the preceding claim, in which the bicarbonate is sodium bicarbonate.

3. Pedicucidal composition according to either one of the preceding claims, in which the particulate bicarbonate is in the form of particles having a particle size such that at least 90% of the said particles have a mean diameter of less than 100 µm.

4. Pedicucidal composition according to the preceding claim, in which the diameter is less than 70 µm.

5. Pedicucidal composition according to any one of the preceding claims, in which the composition additionally comprises at least 1% of silica.

6. Pedicucidal composition according to any one of the preceding claim, in which the composition additionally comprises from 5 to 20% of silica.

7. Pedicucidal composition according to Claims 5 or 6, in which the silica is in the form of particles having a mean diameter of less than 10 µm.

8. Pedicucidal composition according to any one of the Claims 5 to 7, in which the silica is in an amorphous (and non crystalline) form.

9. Pedicucidal composition according to any one of the preceding Claims devoid of any neurotoxic substance.

10. Pedicucidal composition according to any one of the preceding Claims, in the form of a powder, a cream, a gel, an aqueous suspension or an aqueous solution.

11. Pedicucidal composition according to Claim 10 in gel form and comprising gelling polymers.

12. Pedicucidal composition according to Claim 11 in which gelling polymers are polysaccharides, pectin, xanthan.

13. Pedicucidal composition according to any one of the preceding Claims for eliminating the louse Pediculus humanus from human head hair, by bringing the composition into contact with the head hair for at least 1 hour, the operation of bringing into contact for at least 1 hour subsequently being repeated at the earliest two days later.

14. Process for combating the louse Pediculus humanus infesting body linen, bedding and in particular pillows, and cleaning materials, such as towels, according to which the louse is brought into contact with a composition according to any one of Claims 1 to 13.

## Patentansprüche

1. Pedikulizide Zusammensetzung, umfassend mindestens 60 Gew.-% Alkalimetallhydrogencarbonat, zur Verwendung bei der Eliminierung der Laus Pediculus humanus von menschlichem Kopfhaar.

2. Pedikulizide Zusammensetzung nach dem vorhergehenden Anspruch, wobei es sich bei dem Hydrogencarbonat um Natriumhydrogencarbonat handelt.

3. Pedikulizide Zusammensetzung nach einem der beiden vorhergehenden Ansprüche, wobei das teilchenförmige Hydrogencarbonat in Form von Teilchen mit einer solchen Teilchengröße, das mindestens 90% der Teilchen einen mittleren Durchmesser von weniger als 100 µm aufweisen, vorliegt.

4. Pedikulizide Zusammensetzung nach dem vorhergehenden Anspruch, wobei der Durchmesser weniger als 70 µm beträgt.

5. Pedikulizide Zusammensetzung nach einem der vorhergehenden Ansprüche, wobei die Zusammensetzung zusätzlich mindestens 1% Siliciumdioxid umfasst.

6. Pedikulizide Zusammensetzung nach einem der vorhergehenden Ansprüche, wobei die Zusammensetzung zusätzlich 5 bis 20% Siliciumdioxid umfasst.

7. Pedikulizide Zusammensetzung nach Anspruch 5 oder 6, wobei das Siliciumdioxid in Form von Teilchen mit einem mittleren Durchmesser von weniger als 10 µm vorliegt.

8. Pedikulizide Zusammensetzung nach einem der Ansprüche 5 bis 7, wobei das Siliciumdioxid in amorpher (und nichtkristalliner) Form vorliegt.

9. Pedikulizide Zusammensetzung nach einem der vorhergehenden Ansprüche, die frei von jeglicher neurotoxischer Substanz ist.

10. Pedikulizide Zusammensetzung nach einem der vorhergehenden Ansprüche in Form eines Pulvers, einer Creme, eines Gels, einer wässrigen Suspension oder einer wässrigen Lösung.

11. Pedikulizide Zusammensetzung nach Anspruch 10, die in Gelform vorliegt und gelierende Polymere umfasst.

12. Pedikulizide Zusammensetzung nach Anspruch 11, wobei es sich bei den gelierenden Polymeren um Polysaccharide, Pektin, Xanthan handelt.

13. Pedikulizide Zusammensetzung nach einem der vorhergehenden Ansprüche zur Eliminierung der Laus Pediculus humanus von menschlichem Kopfhaar durch Inberührungbringen der Zusammensetzung mit dem Kopfhaar über einen Zeitraum von mindestens 1 Stunde, wobei der Arbeitsgang des Inberührungbringens über einen Zeitraum von mindestens 1 Stunde frühestens zwei Tage später wiederholt wird.

14. Verfahren zur Bekämpfung der Laus Pediculus humanus, die Unterwäsche, Bettwäsche und insbesondere Kopfkissen und Reinigungsmaterialien, wie Handtücher, verseucht, gemäß dem die Laus mit einer Zusammensetzung nach einem der Ansprüche 1 bis 13 in Berührung gebracht wird.

## Revendications

1. Composition pédiculicide comprenant au moins 60% en poids de bicarbonate de métal alcalin pour une utilisation dans l'élimination du pou *Pediculus humanus* des cheveux humains.

2. Composition pédiculicide selon la revendication précédente, **caractérisée en ce que** le bicarbonate est du bicarbonate de sodium.

3. Composition pédiculicide selon l'une ou l'autre parmi les revendications précédentes, **caractérisée en ce que** le bicarbonate particulaire se trouve sous forme de particules ayant une taille de particules telle qu'au moins 90% desdites particules ont un diamètre moyen inférieur à 100 µm.

4. Composition pédiculicide selon la revendication précédente, **caractérisée en ce que** le diamètre est inférieur à 70 µm.

5. Composition pédiculicide selon l'une quelconque des revendications précédentes, **caractérisée en ce que** la composition comprend en outre au moins 1% de silice.

6. Composition pédiculicide selon l'une quelconque des revendications précédentes, **caractérisée en ce que** la composition comprend en outre de 5 à 20% de silice.

7. Composition pédiculicide selon les revendications 5 ou 6, **caractérisée en ce que** la silice se trouve sous la forme de particules ayant un diamètre moyen inférieur à 10 µm.

8. Composition pédiculicide selon l'une quelconque des revendications 5 à 7, **caractérisée en ce que** la silice se trouve sous forme amorphe (et non cristalline).

9. Composition pédiculicide selon l'une quelconque des revendications précédentes, exempte de substances neurotoxiques.

10. Composition pédiculicide selon l'une quelconque des revendications précédentes, sous la forme d'une poudre, d'une crème, d'un gel, d'une suspension aqueuse ou d'une solution aqueuse.

11. Composition pédiculicide selon la revendication 10, sous forme de gel et comprenant des polymères gélifiants.

12. Composition pédiculicide selon la revendication 11, **caractérisée en ce que** les polymères gélifiants sont des polysaccharides, des pectines ou du xanthane.

13. Composition pédiculicide selon l'une quelconque des revendications précédentes, pour éliminer le pou *Pediculus humanus* des cheveux humains, par la mise en contact de la composition avec les cheveux pendant au moins 1 heure, l'opération de mise en contact pendant au moins 1 heure étant ultérieurement répétée au plus tôt deux jours plus tard.

14. Procédé de lutte contre une infestation, par le pou *Pediculus humanus,* du linge de corps, du linge de lit et en particuliers des oreillers, et des matériaux de nettoyage, tels que les serviettes, **caractérisé en ce que** le pou est mis en contact avec une composition selon l'une quelconque des revendications 1 à 13.
